# EUROPEAN PATENT SPECIFICATION

(11) **EP 3 935 149 B1**
(45) Date of publication and mention of the grant of the patent: **23.04.2025**
(21) Application number: 20711087.5
(22) Date of filing: 06.03.2020
(51) Int. Cl.: C12M 1/00, C12M 1/26, C12N 5/00

(54) **METHOD FOR SEPARATING CELLS USING A TEMPERATURE-RESPONSIVE POLYMER**
VERFAHREN ZUR TRENNUNG VON ZELLEN UNTER VERWENDUNG EINES TEMPERATURRESPONSIVEN POLYMERS
PROCÉDÉ DE SÉPARATION DE CELLULES À L'AIDE D'UN POLYMÈRE SENSIBLE À LA TEMPERATURE

(30) Priority: 08.03.2019 DE 102019106011
(43) Date of publication of application: 12.01.2022
(73) Proprietor: Universität Siegen, 57076 Siegen (DE)
(72) Inventor: MÜLLER, Mareike, 57072 Siegen (DE); SCHÖNHERR, Holger, 57258 Freudenberg (DE); JIANG, Siyu, 57076 Siegen (DE); VOSS, Yvonne, 26524 Hage (DE); WASSEL, Ekram, 58840 Plettenberg (DE)
(74) Representative: Maiwald GmbH
(86) International application number: PCT/EP2020/055982
(87) International publication number: WO 2020/182645

(56) References cited:
- EP-A1- 2 574 664
- EP-A1- 3 045 519
- US-A1- 2003 036 196
- US-A1- 2003 219 889

## Description

### FIELD OF THE INVENTION

The invention relates to a specific method for separating cells comprising a specific cell culture substrate comprising a temperature-responsive polymer layer, wherein the temperature-responsive polymer layer exhibits a lower critical solution temperature (LCST) of 10°C to 50°C. Additionally, the invention is directed to a use of a specific cell culture substrate comprising a temperature-responsive polymer layer for cell separation.

### BACKGROUND OF THE INVENTION

For many applications, such as laboratory experiments or the detection of diseases, a cell separation or cell purification step is needed. Most techniques are based on binding cells to a substrate surface and then detaching the cells for further use. It is general practice, for example, to release or detach cells adhered to a cell culture substrate by using protein-digesting enzymes such as trypsin, or by scraping the cells off mechanically, usually manually. However, these techniques can damage the cells or lead to the spontaneous alteration of cell type, such as differentiation of undifferentiated cells.

Further techniques for cell separation or purification, especially in the field of stem cell cultivation, are based on magnetic particles or include chemical agents such as the stem cell technology ReLeSR, which is an enzyme-free reagent for dissociation and passaging of human pluripotent stem cells (hPSCs) to generate optimally sized aggregates. Furthermore, antibody-based methods or micro-RNA-switches are known.

In the last years, polymer-based coatings and materials for gene- and active agent release have come into focus. In many cases, these surfaces can alter their structure or chemical or physical characteristics depending on environmental conditions.

Solid support-based cell culture techniques based on polymer-coated cell culture vessels, in particular based on PNIPAM-coated cell culture vessels and surfaces, are disclosed, for example in JP2006223106A and US201000330674A1. The application US2003219889 describes the use of a photo-sensitive polymer as substrate for cell culture. .

These techniques allow the attachment and detachment of cells and cell sheets from thermo-responsive surfaces.

However, these methods do not provide for separation of different types of cells, or, if they do, cell separation is not sufficiently selective or only feasible with time-consuming, expensive, or cell damaging methods. Another disadvantage of these polymer-based methods is the toxicity of monomers remaining as impurities or the insufficient biocompatibility of polymer layers.

While there is a plurality of methods and techniques available for cell release, cell separation, or cell purification, most of these methods are not applicable for selective cell separation or have disadvantages that need to be overcome. Thus, there is need for separation techniques which allow selective cell separation and/or purification and avoid one or more of the above-mentioned disadvantages of existing techniques.

### SUMMARY OF THE INVENTION

The present invention meets this need by providing a method for separating cells using a cell culture substrate comprising a temperature-responsive polymer layer. At least two different types of cells adhere to said substrate under given medium conditions with at least one type of cells being releasable by modification of said medium temperature.

Preferably, the modified medium conditions include a temperature reduction. Thus, a mild, uncomplicated, selective cell separation method has been developed which meets the need for such a method in the field.

In a first aspect, the invention relates to a method for separating cells, comprising or consisting of the following steps:
(a) providing a sample containing at least two different types of cells;
(b) contacting the sample with a cell culture substrate comprising a temperature-responsive polymer layer, wherein the temperature-responsive polymer layer exhibits a LSCT of 10°C to 50°C;
(c) subjecting the cell culture substrate and the cells to medium conditions which allow the two different types of cells to adhere to the cell culture substrate;
(d) modifying the temperature conditions of step (c) to decrease adherence of at least one first type of cells to the cell culture substrate, wherein the adherence of at least one second type of cells remains essentially unaffected;
(e) optionally flushing the cell culture substrate of step (d) with a suitable cell culture medium or solvent to support the release of the at least one first type of cells with decreased adherence to the cell culture substrate;
(f) optionally agitating the cell culture vessel after step (d) or optionally after step (e) to support the release of the at least one first type of cells with decreased adherence to the cell culture substrate;
(g) separating the at least one first type of cells released from the cell culture substrate in step (d) and/or
(e) and/or (f) from the at least one second type of cells that remain adhered to the cell culture substrate.

In various embodiments, the medium conditions that are modified in step (d) further comprise pH, ionic strength, solvent, cell culture medium, salt, surfactant, and/or electric or magnetic field conditions, and their control and adjustment, as necessary.

In various embodiments, the cell culture substrate and the cells of step (c) are subjected to medium conditions comprising a temperature of 2 °C to 38 °C, preferably of 10 °C to 38 °C, more preferably of 20 °C to 38 °C, more preferably of 28 °C to 38 °C, more preferably of 30 °C to 38 °C or 35 °C to 37.5 °C, most preferably about 37 °C. The cell culture substrate and the cells of step (c) may be subjected to said medium conditions for 1 minute up to 10 days, preferably for 1 hour up to 6 days, more preferably for about 12 hours to about 5 days, about 24 hours to about 48, 72, 96 or 120 hours, in various embodiments, about 24 hours, or about 48 hours, or about 5 days.

In various embodiments, modifying the medium conditions of step (d) comprise a temperature reduction to 2 °C to 36 °C, preferably 10 °C to 30°C. The temperature reduction is, in various embodiments, a reduction by at least 2 °C, preferably by at least 5 °C, more preferably by at least 8 °C, more preferably by at least 12 °C, and even more preferably by at least 15 °C. This may mean that when the original temperature is about 37 °C, the reduced temperature is about 35 °C, about 32°C, about 29 °C, about 25 °C, about 22°C or less. Preferably, the cell culture substrate and the cells of step (d) are subjected to the modified medium conditions for 1 minute up to 10 hours, preferably for 15 minutes up to 6 hours or 30 minutes to 3 hours.

In various embodiments, the cell culture substrate comprises a solid support, preferably coated with a metal. The metal may, in some embodiments, be selected from gold, titanium, or an alloy comprising one or both thereof. The coating is typically on one surface of the solid support material and may cover only parts of the surface, in particular those parts that are intended for later contact with the cells and the cell culture medium.

Preferably, the solid support comprises or consists of a material selected from the group consisting of glass, silicone, polymers, e.g. polystyrene, polymethylmethacrylate, or polycarbonate; ceramic, metals, metal or metalloid oxides, inorganic salts or other solids, or a mixture thereof.

Further preferred, the solid support has any form usable and known in the art for such purposes, comprising micro carriers, multi well plates, micro well plates, membranes, thin films, filters, fibers, flasks, sheets, discs, beads, threads, particles, petri-dishes, plates, fabrics, non-wovens, bottles, tubes, rods, pipes, wafers, nanofiber mats, or foams.

Any of the afore-mentioned solid support forms and materials may be coated with a metal, as described above.

In various embodiments, the temperature-responsive polymer layer is comprised as a coating on at least one surface or surface part of the solid support. The respective part of the solid support surface on which the temperature-responsive polymer layer is present as a coating may be the part covered by a metal coating, as described above, but is typically one of the surfaces or surface parts intended to come into contact with the cells and/or the culture medium.

The binding of the temperature-responsive polymer layer to the solid support, usually in form of a coating, may be facilitated by an initiator compound. Preferably, the initiator compound may comprise a mercapto or silane group coupled to a hydrocarbon backbone that optionally comprises another functional group that allows coupling to the polymer molecules.

Preferably, the initiator comprises or consists of ω-mercaptoundecyl bromoisobutyrate or (2-bromopropan-2-yl4-(trichlorosilyl)butanoate).

For this, the initiator compound is contacted with and bound to surface areas of the solid support onto which the binding of the polymer layer is desired.

In various embodiments, the temperature-responsive polymer layer may be generated *in situ* by using a polymerization technique by which monomers are reacted with the initiator and form polymers directly on the substrate surface. Preferred is polymerization *in situ* by surface-initiating atom transfer radical polymerization starting from an initiator compound coupled to the solid support.

The temperature-responsive polymer layer may comprise homo or copolymers, such as block copolymers, with homopolymers being preferred. In various embodiments, the stimulus-responsive polymer layer comprises, consists essentially of or consists of (meth)acrylate or (meth)acrylamide homo- or copolymers, preferably polymers of (meth)acrylates modified (esterified) with alkylene glycols. One particularly preferred example are homopolymers of esters of (meth)acrylates with poly(ethylene glycol) or capped derivatives thereof, the poly(ethylene glycols) having 2 to 5 ethylene glycol units and the capping being with an alkyl group, preferably methyl or ethyl. Particularly preferred are poly(di(ethylene glycol)methyl ether methacrylate) polymers, in particular the homopolymers thereof.

Preferably, the temperature-responsive polymer layer has a dry ellipsometric thickness of 1 to 1000 nm, more preferably of 1.5 to 100 nm, more preferably of 2 to 25 nm, more preferably of 3 to 10 nm, most preferably of about 5 nm, preferably at 25 °C measured by an ellipsometer in air, atomic force microscopy (AFM), interferometry, surface plasmon resonance (SPR), X-Ray Photoelectron Spectroscopy (XPS), and/or Fourier-Transform Infrared Spectroscopy (FTIR).

The temperature-responsive polymer (layer) exhibits an (apparent) lower critical solution temperature (LCST) of 10 to 50 °C, preferably of 12 to 45 °C, more preferably of 15 to 40 °C, more preferably of 20 to 37 °C, more preferably of 25 to 36 °C, most preferably of 32 to 35 °C, preferably in a suitable cell culture medium.

The cells to be separated may be mixtures of
(i) different cell lines, preferably tumor cells and healthy cells, such as fibroblast cells; or
(ii) pathologically altered cells and normal cells; or
(iii) differentiated cells and undifferentiated stem cells, preferably mixtures of undifferentiated human induced pluripotent stem cells and differentiated cells; or
(iv) different cell types derived from a certain tissue (e.g. from biopsies).

In various embodiments, the cells to be separated are added to the cell culture substrate in amounts of 1,000 to 100,000 cells/cm², preferably of 10,000 to 40,000 cells/cm², more preferably of 15,000 to 20,000 cells/cm².

In another aspect, the invention relates to the use of a cell culture substrate comprising a stimulus-responsive polymer layer as defined in the embodiments according to the invention, for cell separation.

### BRIEF DESCRIPTION OF THE DRAWINGS

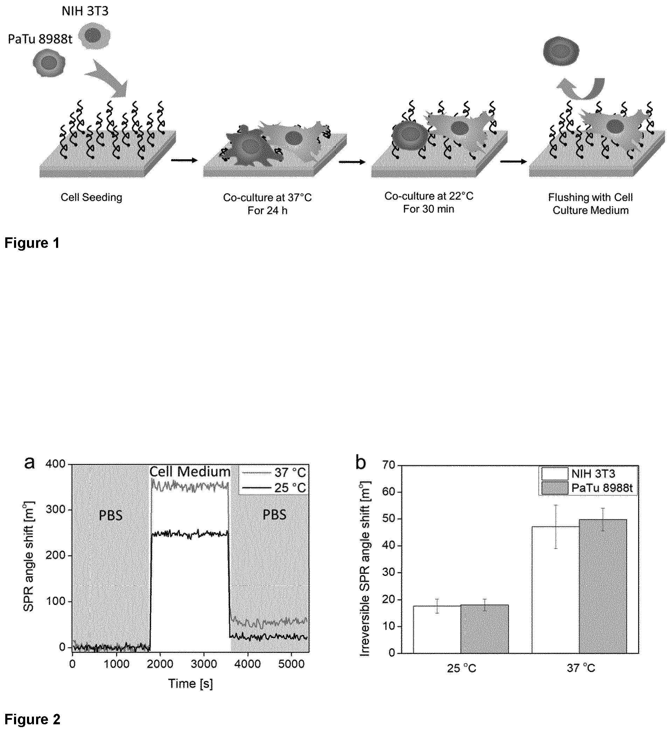

**Figure 1****.** Co-culture of PaTu 8988t and NIH 3T3 cell lines on a 5 nm thick poly(di(ethylene glycol)methyl ether methacrylate) (PDEGMA) layer. Cell attachment and proliferation of both cell lines occur on PDEGMA at 37 °C. Only PaTu 8988t cells show substantial changing on cell morphology and weaker cell adherence when the environment temperature is reduced from 37 °C to 22 °C for 30 min. Then, a selective cell detachment of PaTu 8988t is obtained at 22 °C by flushing with cell medium.
**Figure 2(a)** Kinetic surface plasmon resonance (SPR) data for NIH 3T3 cell culture medium adsorption on 5 nm PDEGMA at 25 °C and 37 °C. (b) Irreversible SPR angle shift of different cell culture medium adsorption on 5 nm PDEGMA at 25 °C and 37 °C (NIH 3T3: white, PaTu 8988t: grey).

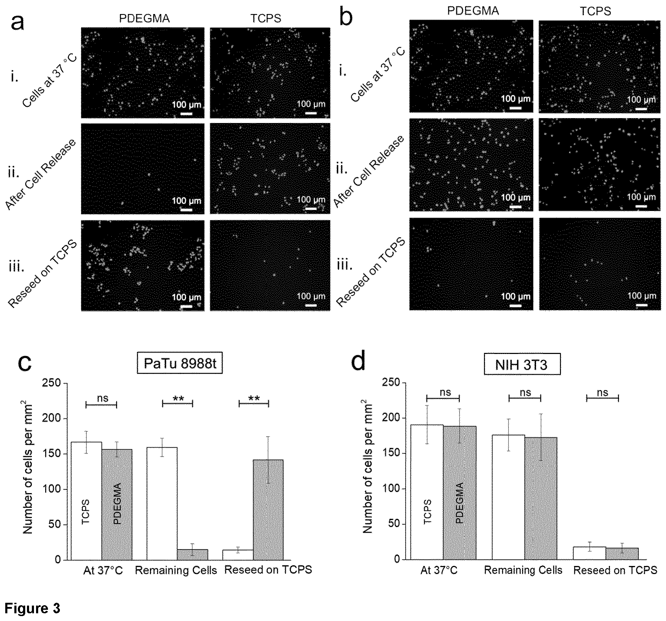

**Figure 3****.** Fluorescence microscopy images of (a) PaTu 8988t and (b) NIH 3T3 cells on 5 nm PDEGMA and TCPS in different states: (i) after 24 h cultivation at standard conditions (37 °C, 5 % CO₂); (ii) after cell releasing at 22 °C, the remaining cells of PaTu 8988t and NIH 3T3 on the substrates; (iii) the released cells were reseeded on new TCPS dishes and incubated for 2 h at 37 °C. The cell nuclei were stained with Hoechst 33258 in blue. Quantitative evaluation of (c) PaTu 8988t and (d) NIH 3T3 cell number at 37 °C after 24 h cultivation and after cell medium washing step on the substrates as well as detached cells reseeding on new TCPS dishes (grey: TCPS, white: 5 nm PDEGMA), where error bars are standard error (n = 3), ns means not significant, indicating p > 0.05, * p < 0.05, and ** p < 0.0001.

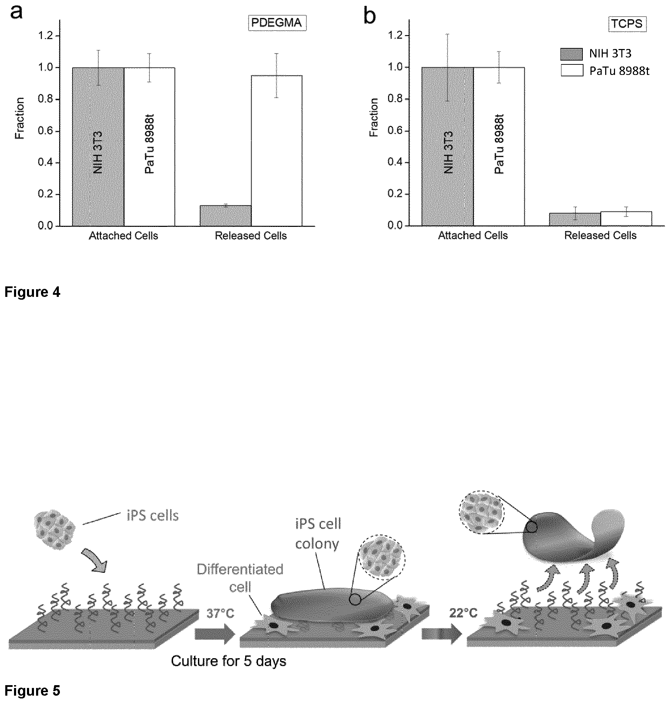

**Figure 4****.** Statistical analysis of NIH 3T3 (grey) and PaTu 8988t (white) co-cultures on (a) 5 nm PDEGMA layer and (b) TCPS in the attached and the released state. PaTu 8988t cells were stained with Vybrant^{®} DiD (red), and NIH 3T3 cells were labelled with Vybrant^{®} DiO (green). The samples were quantitatively analyzed by fluorescence microscopy. The error bars are the standard deviation from 3 different images per condition. According to Figure 4, where the fraction of two cell lines in the different states on PDEGMA and TCPS are compared, there was a significant difference in the composition of the co-culture. There were 95 ± 6 % of PaTu 8988t cells detached from PDEGMA layer (Figure 4a), whereas only few cells were released from TCPS (9 ± 2 %, Figure 4b). By contrast, a small amount of NIH 3T3 fibroblasts detached from PDEGMA and TCPS surfaces, 13 ± 1 % and 8 ± 4 %. The observations confirm that 5 nm thin PDEGMA layers enable to release PaTu 8988t cells preferentially from a co-culture with NIH 3T3 cells and thus afford a strategy for selective cell detachment and thus purification.
**Figure 5****.** iPS cells seeded on PDEGMA brushes. Cells grow into cell colony in 5 days (incubation at 37 °C). The undifferentiated iPS cell colony detaches from the heterogeneous culture, with the differentiated cells on PDEGMA brushes at 22 °C.

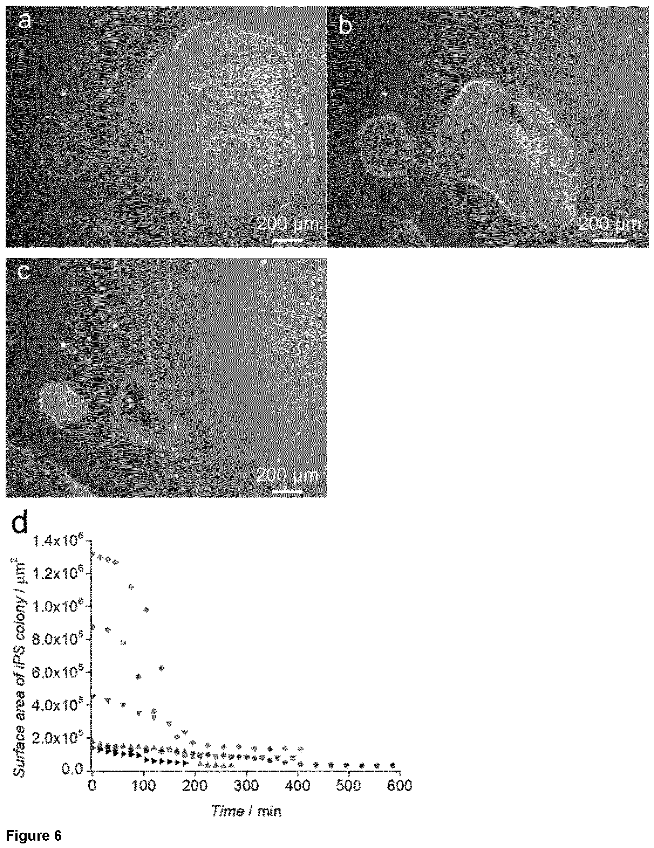

**Figure 6****.** Optical microscopy images of iPS cells cultured: (a) in colonies on 5 nm PDEGMA brushes for 5 days at 37 °C, detachment of iPS cell colonies from 5 nm PDEGMA brushes at 22 °C after (b) 135 min, and after (c) 345 min. (d) Time needed for reducing surface area of different iPS colonies on 5 nm PDEGMA brushes at 22 °C.

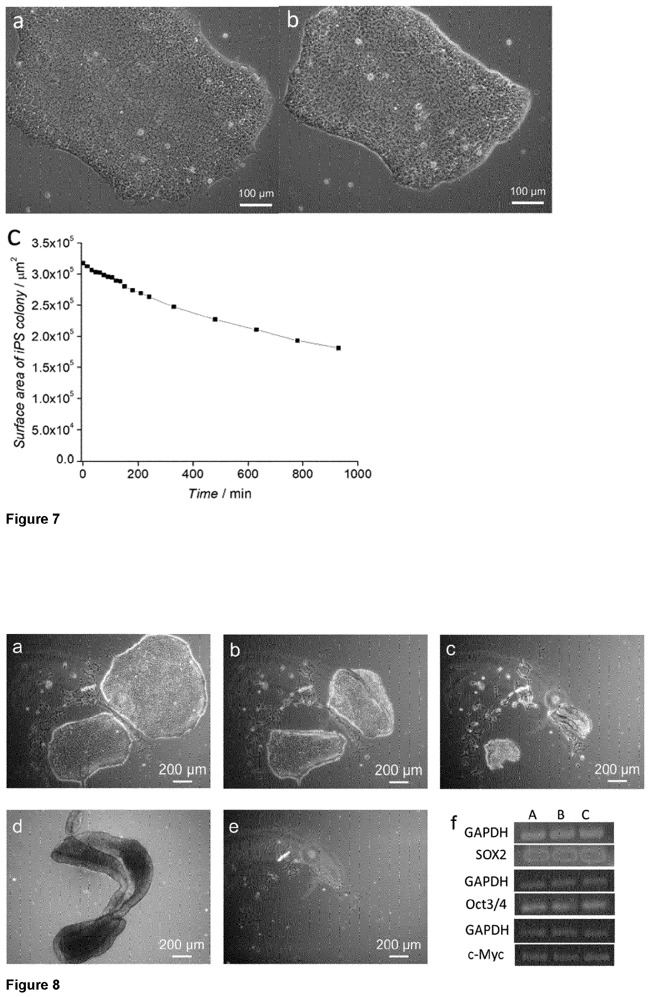

**Figure 7(a)** iPS colony on matrigel at 37 °C. (b) Morphology changing of the iPS colony on matrigel at 22 °C after 360 min. (c) Time needed for reducing surface area of different iPS colonies on matrigel at 22 °C.
**Figure 8****.** Optical microscopy images of iPS cells cultured: (a) in colonies with surrounding differentiated cells on 5 nm PDEGMA layer for 5 days at 37 °C, selective detachment of undifferentiated iPS cell colonies from 5 nm PDEGMA layer at 22 °C after (b) 120 min, and after (c) 210 min. (d) Released iPS cell colonies floating in the cell culture medium after knocking the culture plate. (e) Remaining differentiated cells on the substrate after replacing the culture plate at 37 °C for 60 min. (f) The semi-quantitative reverse transcriptase PCR of iPS cells cultured on (A) matrigel and (B) 5 nm PDEGMA for 5 days, (C) released iPS cells from PDEGMA layer by the thermal stimulus. The gene expression level of pluripotency markers Sox 2, Oct 3/4, c-Myc confirmed the pluripotency maintenance of iPS cells grown on 5 nm PDEGMA as well as released iPS cells compared to the standard growth conditions on matrigel. GAPDH as a housekeeping gene serves as an internal reference gene for normalization.

### DETAILED DESCRIPTION OF THE INVENTION

The following detailed description refers to, by way of illustration, specific details and embodiments in which the invention may be practiced. These embodiments are described in sufficient detail to enable those skilled in the art to practice the invention. Other embodiments may be utilized and structural and logical changes may be made without departing from the scope of the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. The singular terms "a", "an" and "the" include plural referents unless context clearly indicates otherwise. Similarly, the word "or" is intended to include "and" unless the context clearly indicates otherwise. The term "comprises" means "includes". In case of conflict, the present specification, including explanations of terms, will control.

The terms "one or more" or "at least one", as interchangeably used herein, relate to at least 1, or at least 2, 3, 4, 5, 6, 7, 8, 9, 10, 11, 12, 13, 14, 15, 20, 25 or a plurality of species, e.g. cells. In this connection, the term "plurality" means more than one, preferably 2 or more, such as up to 1000.

Numeric values specified without decimal places here refer to the full value specified with one decimal place, i.e. for example, 99 % means 99.0 %, unless otherwise defined.

The terms "about" or "approximately" or "approx.", in connection with a numerical value, refer to a variance of ±10 %, preferably ±5 %, more preferably ±2 %, more preferably ±1 %, and most preferably less than ±1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

"Essentially", for example in "consists essentially of" typically means that the composition or layer may comprise little amounts of further components in addition to the mentioned component. Preferably, the further components are comprised in amounts of less than 1 wt.-%, preferably less than 0.1 wt.-%, more preferably less than 0.01 wt.-%, more preferably less than 0.001 wt.-%, wherein most preferably the composition or the layer is free of further components in addition to the mentioned component(s), if not explicitly stated otherwise.

The method for separating cells according to the invention, comprising or consisting of the following steps:
(a) providing a sample containing at least two different types of cells;
(b) contacting the sample with a cell culture substrate comprising a temperature-responsive polymer layer, wherein the temperature-responsive polymer layer exhibits an LCST of 10°C to 50°C;
(c) subjecting the cell culture substrate and the cells to medium conditions which allow the two different types of cells to adhere to the cell culture substrate;
(d) modifying the temperature conditions of step (c) to decrease adherence of at least one first type of cells to the cell culture substrate, wherein the adherence of at least one second type of cells remains essentially unaffected;
(e) optionally flushing the cell culture substrate of step (d) with a suitable cell culture medium or solvent to support the release of the at least one first type of cells with decreased adherence to the cell culture substrate;
(f) optionally agitating the cell culture vessel after step (d) or optionally after step (e) to support the release of the at least one first type of cells with decreased adherence to the cell culture substrate;
(g) separating the at least one first type of cells released from the cell culture substrate in step (d) and/or
(e) and/or (f) from the at least one second type of cells that remain adhered to the cell culture substrate.

Preferably, the steps of the method according to the invention are carried out in the described order.

The term "separating cells" means that at least two different types of cells contained in a sample are (spatially) separated by the method according to the invention. However, the term shall not include information on the purity or completeness of the cell separation, if not explicitly stated otherwise. Therefore, it is included in the scope of the invention that some of at least one type of cells to be separated from the at least one other type of cells may remain on the cell culture substrate and are thus not separated from the at least one other type of cells. In such circumstances, the term "separation" is thus tantamount to an enrichment of at least one type of cells by reduction of the fraction of at least one other type.

In a preferred embodiment, at least one type of cells contained in the sample of step (a) is separated by the method according to the invention by at least 50 %, more preferably by at least 60 %, more preferably by at least 70 %, more preferably by at least 80 %, more preferably by at least 90 %, more preferably by at least 95 %, most preferably by at least 99 %. These percentages relate to the number of the cells to be separated relative to the total number of cells obtained, i.e. the percentage of the desired cell type in the cell population obtained in the separation step. Alternatively, the separation may result in an enrichment of the cells to be separated that is expressed in an increase of the fraction of the desired cells by at least 50 %, at least 100 % or more. For example, if half of the cells in the starting mixture are the desired cells, an enrichment by 50 % would mean a final fraction of 75 % (i.e. 75 % of the cells of the obtained cell population after the separating step are the desired cells), while under the same conditions an enrichment of 100 % would mean a final fraction of 100 %. In any case, the separation preferably leads to an increase of the number of cells to be separated relative to the total number of cells (the fraction of the cells to be separated) by at least 10 %, more preferably by at least 20 %, even more preferably by 30 %, 40 %, 50 % or more. It is preferred that after the separation the desired cell type makes up at least 50 % of the remaining cell population, more preferably at least 70 %. The desired cell type may be the one released in the separation step or the one that remains adhered to the substrate.

The term "cell" or "cells" or "cell lines" refers to any type of cell(s) that can be cultured in a cell or tissue culture vessel. Unless otherwise specified, any (living) organism, appropriate for methods and uses according to the invention, can be used to create cells or for sampling of cells. In various embodiments, the organism is a single cell organism or a multicellular organism, preferably a multicellular organism. In a preferred embodiment, the sample containing at least two different types of cells results from sampling from a human, mammalian, or animal individual. In various, preferred embodiments, the organism is mammalian, including a human.

Furthermore, the sample containing at least two different types of cells may result from cell experiments, e.g. from cell culturing or other cell purification methods.

In other embodiments, the sample containing at least two different types of cells may be from a natural source, e.g. may be a biological sample, such as blood, tissue and the like, or natural environmental sources like water, wood, or soil.

In various embodiments, the cells are selected from the group consisting of, but not necessarily limited to, fibroblasts, myoblasts, myotube cells, corneal cells, vascular endothelial cells, smooth muscle cells, cardiomyocytes, dermal cells, epidermal cells, mucosal epithelial cells, mesenchymal stem cells, ES cells, iPS cells, osteoblasts, osteocytes, chondrocytes, fat cells, neurons, hair root cells, dental pulp stem cells, ß-cells, hepatocytes, glial cells, adipocytes, (small intestine) epithelial cells, epidermal keratinocytes, and combinations thereof.

Further suitable cells are bone marrow derived stem cells, bone marrow derived stroma cells, adipose-derived stem cells, adipose-derived stromal cells, hematopoietic stem cells, fibroblasts, cell lines, neuroblastoma cells, Chinese hamster ovary cells, and human embryonic kidney cells.

In preferred embodiments, cells to be separated are mixtures or compositions of
(i) different cell lines; or
(ii) pathologically altered cells and normal cells; or
(iii) differentiated stem cells and undifferentiated stem cells; or
(iv) different cell types derived from a certain tissue (e.g. from biopsies).

Typically, the term "normal cells", as used in the context of this application, means healthy, in particular (pathologically) non-altered cells. In contrast, "pathologically altered cells" include non-healthy cells, such as cancer cells, spontaneous differentiated cells, or any other cell type, which can be altered due to a disease or medical condition, if not explicitly stated otherwise.

In particular, the invention is relevant for cell culturing, generation, and purification of stem cells, applications relating to pathology/histology, and testing of biopsy samples (e.g. for cancer diagnostics), or for preparation of skin models for (biological) product safety tests.

In one embodiment, the sample containing at least two different types of cells is a mixture of different cell lines, for example tumor cells such as (pancreatic) carcinoma cells, and fibroblast cells.

In another, preferred embodiment, the sample containing at least two different types of cells is a mixture of undifferentiated human induced pluripotent stem cells and differentiated cells.

In a preferred embodiment, the sample containing at least two different types of cells is a mixture of different cell types from a certain tissue, e.g. from biopsies.

Suitable tissues, without being limited to it, may be skin or epithelial tissue, connective or supporting tissue, cartilage tissue, bone (marrow) tissue, muscle tissue, nerve tissue or tumor tissue.

The method according to the invention may provide a mild, non-invasive, time-saving technique for selective cell separation or cell release depending on their differentiation state or cell type.

In a preferred embodiment, the method according to the invention is used to speed up the pathological examination of cells in samples of body tissues or bodily fluids, including but not limited to blood, saliva, semen, and the like.

This method may also help to separate undifferentiated stem cells from spontaneous differentiated stem cells in the field of stem cell production, wherein the spontaneous differentiated stem cells represent contaminations of the sample.

The terms "stimulus-responsive polymer layer" and "stimulus-responsive polymer coating" are used interchangeably in this application.

The stimulus-responsive polymer layer is a thermo-responsive polymer layer, wherein the temperature-responsive layer exhibits a LSCT of 10°C to 50°C. In the following description pages, stimulus-responsive polymer layer always refer to such a temperature-responsive polymer layer.

Suitable synthetic polymers are, for example, selected from the group of poly(N-isopropyl acrylamide) (PNIPAM), poly-2-(2-methoxyethoxy) ethyl methacrylate-co-oligo(ethylene glycol) methacrylate (poly(MEO₂MA-*co*-OEGMA)), poly(di(ethylene glycol)methyl ether methacrylate) (PDEGMA), poly(oligo(ethylene glycol)methyl ether methacrylate) (POEGMA), PNIPAM-*co*-POEGMA, POEGMA-*co*-PDEGMA, poly(MEO₂MA₅₀-*co*-HOEGMA₂₀-*co*-HEMA₃₀), poly(dimethylaminoethyl methacrylate) (PDMAEMA) and poly[tri-(ethylene glycol) ethyl ether methacrylate] (P(TEGMA-EE)).

In various embodiments, the at least one synthetic polymer comprises, consists essentially of or consists of at least one homopolymer and/or at least one copolymer. In a preferred embodiment, the at least one synthetic polymer comprises or consists of at least one homopolymer, more preferably one single homopolymer.

In particular, the at least one synthetic polymer comprises, consists essentially of or consists of at least one thermo-responsive homopolymer, preferably of at least one (meth)acrylate or (meth)acrylamide homo- or copolymer, more preferably a polymer of (meth)acrylates modified (esterified) with alkylene glycols, even more preferably at least one homopolymer of esters of (meth)acrylates with poly(ethylene glycol) or capped derivatives thereof, most preferably at least one poly(di(ethylene glycol)methyl ether methacrylate) homopolymer.

In a preferred embodiment, the temperature-responsive polymer layer is not cytotoxic.

The temperature-responsive polymer layers of the invention formed by synthetic polymers are also known under the term "polymer brushes", end-grafted or grafted polymer layers and "polymer mushrooms". Polymer brushes are mostly linear polymer chains grafted with one end to a surface (of a solid carrier), with the polymer chain extending outward from the surface. If multiple such polymer chains are grafted to the surface with high grafting density, the polymer chains align parallel to each other and in a schematic representation, such surface structure has the look of a brush. In general, at reduced grafting density polymer mushrooms are being formed.

Stimulus-responsive polymers or polymer layers may allow adhesion and proliferation of cells and thus, the culturing of single cells or cell sheets on the cell culture substrate. Due to their responsive behavior, it is possible to bind and/or release cells or (confluent) cell sheets from cell culture substrates comprising the stimulus-responsive polymer layer by a simple modification of temperature.

The temperature change (typically a temperature reduction) may cause a spontaneous detachment of at least one first type of cells from the cell culture substrate, wherein the adherence of at least a second type of cells remains essentially unaffected. Depending on the external stimulus, the polymer may undergo a structural change or phase transition due to mediated changes in the hydrophilic and hydrophobic interactions around its lower critical solution temperature (LCST).

The lower critical solution temperature (LCST) can be defined as a temperature below which a polymer is fully miscible/soluble in the medium/solvent used. Upon increase above this temperature, miscibility/solubility decreases such that at least partial immiscibility/insolubility occurs. The LCST is typically dependent on the polymer and medium or solvent used.

According to the invention, the temperature-responsive polymer layer exhibits an (apparent) lower critical solution temperature (LCST) of 10 to 50°C, preferably of 12 to 45°C, more preferably of 15 to 40°C, more preferably of 20 to 37 °C, more preferably of 25 to 36 °C, and most preferably of 32 to 35 °C, preferably in a suitable cell culture medium, which is typically an aqueous medium.

In many instances, the observed macroscopic effect can be attributed to a change in the polymer chain from a hydrophobic to a more hydrophilic nature at their switching / volume phase transition conditions, e.g. by temperature change.

For example, appropriately applied environmental stimuli may induce swelling of the polymer chain. The swelling-induced deformation of the polymer chains of the stimulus-responsive polymer layer, can in turn assert mechanical strain upon the cells attached to the cell culture substrate, resulting in the release of cells. In some embodiments, the cell release from the stimuli-responsive polymer layer of the cell culture substrate takes 1 to 60 seconds after the conformational change of the polymer has been induced.

The stimulus change is preferably employed to safely release or harvest cells or cell sheets grown on the stimulus-responsive polymer layer on the cell culture substrate. In the hydrophobic state of the stimulus-responsive polymer layer, the cell culture substrate generally provides more favorable conditions for cell attachment and growth thereon. As compared to the use of proteolytic enzymes (e.g. trypsin) or mechanical scraping, the use of stimulus-responsive polymer layers to release cells or (confluent) cell sheets minimizes damage to cells and their extracellular matrix (ECM), thus preserving their biological functions.

In various embodiments, the thickness or grafting density of the temperature-responsive polymer layer may be a contributing factor to successfully culture cells and attach and/or detach cells and/or cell sheets to/from the cell culture substrate. For example, a higher grafting density typically results in a stretching of the polymers of the temperature-responsive polymer layer. Thus, small differences in cell adherence of different cell types can be used for fast and selective cell separation.

The synthetic polymers of the stimulus-responsive polymer layer of the method according to the invention respond to a change in temperature by undergoing conformational changes. This temperature dependence can be finely tuned and makes thermo-responsive polymers attractive as coatings for cell culture applications.

In preferred embodiments, the temperature-responsive polymer layer, or the synthetic polymers of the temperature-responsive polymer layer, respectively,
(1) comprise or consist of homopolymers, preferably selected from the group consisting of methacrylate homopolymers, such as those defined above, more preferably poly(di(ethylene glycol)methyl ether methacrylate) homopolymers; and/or
(2) have a dry ellipsometric thickness of 1 to 1000 nm, preferably of 1.5 to 100 nm, more preferably of 2 to 25 nm, more preferably of 3 to 10 nm, most preferably of about 5 nm, preferably at 25 °C measured by an ellipsometer in air, atomic force microscopy (AFM), interferometry, surface plasmon resonance (SPR), X-Ray Photoelectron Spectroscopy (XPS), and/or Fourier-Transform Infrared Spectroscopy (FTIR); and
(3) exhibit an (apparent) lower critical solution temperature (LCST) of 10 to 50 °C, preferably of 12 to 45 °C, more preferably of 15 to 40 °C, more preferably of 20 to 37 °C, more preferably of 25 to 36 °C, most preferably of 32 to 35 °C, preferably in a suitable cell culture medium.

In various embodiments, the cell culture substrate comprises a solid support.

The solid support generally may comprise or consist of a material selected from the group consisting of glass, silicone, polymers, e.g. polystyrene, polymethylmethacrylate, or polycarbonate; ceramic, metals, metal or semiconductor oxides, inorganic solids, or a mixture thereof.

Preferably, the solid support is coated with a substance comprising or consisting of a metal, wherein the metal is preferably selected from gold, titanium, or an alloy containing one or both of the afore-mentioned metals.

In order to coat polymers, or rather the temperature-responsive polymer layer, onto the solid support of the cell culture substrate with control of the resulting layer thickness, different approaches are commonly used. Generally, these can be classified into grafting-from, grafting-to, and grafting-through processes.

In the grafting-from approach, monomers are polymerized *in situ* starting from the solid support. Here, radical polymerizations like the electron beam polymerization (EBP), atom-transfer radical polymerization (ATRP), the plasma polymerization and the UV-initiated polymerization are the most commonly used methods.

In various embodiments, suitable initiators for surface-initiated polymerization (grafting-from) use free radical, controlled radical, anionic, cationic, ring opening polymerization methods, especially atom transfer radical (ATRP) polymerization, reversible addition-fragmentation chain transfer (RAFT) polymerization, nitroxide-mediated radical polymerization (NMP), and INIFERTER polymerization.

In the grafting-to approach, an already formed polymer chain, typically, adsorbs onto the surface of the (metal-coated) solid support out of the surrounding solution or medium. This adsorption can be mediated by an activator or connecting compound, which connects the already formed polymer chain with the (metal-coated) solid support. However, every suitable method, known to the person skilled in the art, can be used for the generation of the temperature-responsive polymer layer according to the invention.

In various embodiments, the temperature-responsive polymer layer is alternatively generated by coupling end- or side-group modified temperature-responsive polymers to said solid support (grafting-to). The solid support may contain atoms and / or functional groups, e.g. via a deposited layer of molecules or polymers or functional groups introduced by a treatment (in gas or liquid phase or by plasma), or those atoms and / or functional groups that are inherently present on the surface.

In other embodiments, the temperature-responsive polymer layer is alternatively generated by the grafting-through approach or surface chemical reactions, whereby suitable monomers are attached to a surface and these monomers may be incorporated into a polymer chain synthesized in solution during chain growth.

In various embodiments, the binding of the temperature-responsive polymer layer to the solid support is mediated by activating the solid support with a suitable activator. This activator may be an initiator compound that is, in a first step, reacted with the solid support or the coating of the solid support, such as the above-described metals, and in a second step allows attachment of the polymer layer or generates an active center from which polymerization of the monomers to generate the polymer chain starts.

In a preferred embodiment, an initiator compound facilitates the binding of the temperature-responsive polymer layer to the (coated) solid support.

The initiator may also be bound to an interlayer, comprising or consisting of atoms and / or functional groups, e.g. via a deposited layer of molecules or polymers or functional groups introduced by a treatment (in gas or liquid phase or by plasma), or those atoms and / or functional groups that are inherently present on the surface. The interlayer may be bound to or coated onto the (coated) solid support.

Initiator compounds or systems, usable in the polymer generation, are, for example, peroxides, aliphatic azo compounds, organometallic compounds, or metallic halides, e.g. triethylaluminum or titanium tetrachloride. Generally, such initiator compounds have the functionality to bind to the solid support and to bind to or start formation of the polymer.

Suitable ATRP initiators for the temperature-responsive polymer layer generation are, for example, without being limited to it, 2-azidoethyl 2-bromoisobutyrate, bis[2-(2'-bromoisobutyryloxy)ethyl]disulfide, ω-mercaptoundecyl bromoisobutyrate, (2-bromopropan-2-yl 4-(trichlorosilyl)butanoate), 2-bromoisobutanoic acid N-hydroxysuccinimide ester, 2-bromoisobutyric anhydride, 2-hydroxyethyl 2-bromoisobutyrate, 1,1,1-tris(2-bromoisobutyryloxymethyl)ethane, poly(ethylene glycol) methyl ether 2-bromoisobutyrate, poly(ethylene glycol) bis(2-bromoisobutyrate), 1-(phthalimidomethyl) 2-bromoisobutyrate, or α-bromoisobutyryl bromide. These compounds may be further modified to comprise a functional group that allows attachment to the solid support, for example, in case the solid support is made of metal or coated with a metal, a mercapto or silane group.

In some embodiments, where metal coatings are used for the solid support, the initiator compound may thus comprise or may be ω-mercaptoundecyl bromoisobutyrate or (2-bromopropan-2-yl 4-(trichlorosilyl)butanoate).

In various embodiments, the initiator compound is applied onto the solid support in a mixture with a non-initiating compound. The non-initiator compound may be similarly able to bind to the solid support, but may lack the functionality to initiate polymerization or facilitate polymer binding to the surface. As the density of the initiator may affect polymer chain density on the surface, control of the fraction of the non-initiator allows control of the initiator density. Preferably, the initiator compound is contained in the mixture with the non-initiating compound in a fraction of >0.1, more preferably >0.2, more preferably >0.3, more preferably >0.4, more preferably >0.5, more preferably >0.6, more preferably >0.7, more preferably >0.8, more preferably >0.9, most preferably >0.95.

In one embodiment, the initiator compound is contained in the mixture with the non-initiating compound in a fraction of >0.6.

The higher the fraction of the initiating compound to the non-initiating compound in the mixture, the higher is the density of the initiator compound coupled onto the solid support. Preferably, as stated above, the density of the initiator compound on the solid support determines the grafting density of the synthetic polymer chains bound to the solid support.

In a preferred embodiment, the initiator compound is not mixed with a non-initiating compound, before being applied onto the solid support.

Preferably, the temperature-responsive polymer layer is generated *in situ* by surface-initiating atom transfer radical polymerization starting from an initiator compound coupled to the solid support.

In a preferred embodiment, the initiator compound activates di(ethylene glycol)methyl ether methacrylate (DEGMA) monomers (and/or other acrylate-type monomers) to form a polymer.

In a preferred embodiment, the cell culture substrate comprises a (coated) solid support, preferably a metal-coated solid support, preferably (borosilicate) glass coated with gold, titanium, or a mixture thereof;
and/or
the (coated) solid support is covered with an initiator compound, preferably with a monolayer of the initiator compound, more preferably with ω-mercaptoundecyl bromoisobutyrat or (2-bromopropan-2-yl 4-(trichlorosilyl)butanoate), or a mixture of ω-mercaptoundecyl bromoisobutyrate or (2-bromopropan-2-yl 4-(trichlorosilyl)butanoate) and a non-initiating compound, preferably a mixture of ω-mercaptoundecyl bromoisobutyrate and 16-mercaptohexadecanoic acid (MHDA);
   and/or
the initiator compound connects the (coated) solid support and the stimulus-responsive polymer layer of the cell culture substrate.

Additionally, the cell culture substrate, in particular the temperature-responsive polymer layer or the solid support, can be subjected to treatment by crosslinking or immobilization of functional groups or biomolecules that facilitate the binding of cells to the cell culture substrate.

In various embodiments, the surface of the solid support or the surface of the metal-coated solid support is treated with an appropriate substance, for example fibronectin or collagen, to facilitate attachment of cultured cells onto the cell culture substrate. Additional substances suitable for preparing the cell culture substrate of the method according to the invention are well known to a person of ordinary skill in the art and such embodiments are within the scope of the invention.

In various embodiments, the solid support is part of or incorporated into a cell or tissue culture vessel, or the solid support is the cell or tissue culture vessel. In other various embodiments, the cell culture substrate comprising a solid support is located in a cell or tissue culture vessel and the solid support is not part of the cell or tissue culture vessel.

The term "cell culture vessel" or "tissue culture vessel" refers to any vessel that can be used to grow cells or cell lines. The term "cell culture vessel" is synonymous to "tissue culture vessel". Suitable cell culture vessels include standard vessels (such as petri dishes, cell culture dishes, multi-well plates, and tissue culture flasks), non-adherent vessels (such as blood bags or other cell culture bags), and larger volume and bioreactor systems (such as spinner flasks, roller bottles, stirred-suspension bioreactors, rotating wall vessels, wave bioreactors, and parallel plate, hollow-fiber, fixed bed and fluidized bed systems, and fermentation vessels). A non-exhaustive list of tissue culture vessels includes dishes, flasks, tubes, bottles, bioreactors, and/or microscope slides. Any vessel used to grow cells or cell lines, as known by those skilled in the art, are usable in this invention.

In various embodiments, the solid support, in particular, if the solid support is not part of the cell culture vessel or tissue culture vessel, can have any form known, usable or suitable in this field or for these type of applications, including, but not limited to, micro carriers, multi well plates, micro well plates, membranes, thin films, filters, fibers, flasks, sheets, discs, beads, threads, particles, petri-dishes, plates, fabrics, non-wovens, bottles, tubes, rods, pipes, wafers, nanofiber mats, or foams.

In various embodiments, the solid support may have pores and surface channels, grooves, microstructures, controlled surface topography, controlled porosity, and/or nanostructures, preferably ranging from 5 nm to 10 µm in diameter or width.

In preferred embodiments, the cell culture substrate is present in a micro- or multi-well plate or in a culture dish. The micro- or multi-well plate or culture dish can be covered by a lit, slide, plate or foil, preferably by a glass or plastic lit, slide, plate or foil. Preferably, one cell culture substrate is contained in one well of the micro- or multi-well-plate, or in the culture dish. More preferably, the cell culture substrate is surrounded by a liquid cell culture medium, which is preferably suitable for the cells to be separated. Preferably, the liquid cell culture medium is an aqueous cell culture medium.

In preferred embodiments, the cells added to the cell culture substrate in step (b) are present in a cell culture medium or solvent.

In various embodiments, the cell culture medium is serum free cell culture medium.

Preferably, the cells to be separated are added to the cell culture substrate in amounts of 1,000 to 100,000 cells/cm², more preferably of 10,000 to 40,000 cells/cm², most preferably of 15,000 to 20,000 cells/cm².

In various embodiments, the reaction volume is 100 µL to 10 L, preferably 1 mL to 1 L or to 100 mL or to 50 mL, more preferably about 1 to 10 mL. However, bigger or smaller reactions volumes are also encompassed by the scope of the invention.

In various embodiments, the method according to the invention may comprise a further cell adhesion agent. The term "cell adhesion agent" refers typically to any agent that assists in binding a cell to another cell or surface. A cell adhesion agent can be biological or non-biological. For example, a non-exhaustive list of cell adhesion agents includes arginylglycylaspartic acid peptides, cadherins, integrins, selectins, extracellular matrix, and/or synthetic polymers, wherein the synthetic polymers are different to the synthetic polymers used in the stimulus-responsive polymer layer of the method according to the invention.

In a preferred embodiment, the cell culture substrate and the cells of step (c) of the method according to the invention are subjected to medium conditions comprising a temperature of 2 °C to 38 °C, preferably of 10 °C to 38 °C, more preferably of 20 °C to 38 °C, more preferably of 28 °C to 38 °C or 30 °C to 38 °C, more preferably of 35 °C to 37.5 °C, most preferably about 37 °C.

In various, preferred embodiments, the external stimulus is the temperature.

Preferably, the cell culture substrate and the cells of step (c) of the method according to the invention are subjected to said medium conditions for 1 minute up to 10 days, preferably for 1 hour up to 6 days, more preferably for about 12 hours to about 5 days, about 24 hours to about 48, 72, 96 or 120 hours, in various embodiments, about 24 hours, or about 48 hours, or about 5 days.

In various preferred embodiments, modifying the medium conditions of step (d) of the method according to the invention comprises or consists of a temperature reduction to 2 °C to 36 °C, preferably to 15 °C to 26 °C, more preferably to 22 °C. If the polymer is temperature-sensitive, the temperature change from step (c) to step (d) is typically at least 2 °C, preferably at least 5 °C, more preferably at least 8 °C or more. In preferred embodiments, the temperature change can be as much as 10 °C, 12 °C, 14 °C, 15 °C or more. In a preferred embodiment, the temperature change from step (c) to step (d) is at least 15 °C or about 15 °C. Most preferably, the temperature is changed from (about) 37 °C in step (c) to (about) 22 °C in step (d).

Preferably, the cell culture substrate and the cells are subjected to said modified medium conditions in step (d) for 1 second up to 10 hours, more preferably for 1 minute up to 10 hours, more preferably for 15 minutes up to 6 hour or 30 minutes to 3 hours. In some embodiments, the at least one first type of cells detach from the stimulus-responsive polymer layer of the cell culture substrate within 1 to 60 seconds, after the conformational change of the polymer dependent on the modified medium conditions.

In a preferred embodiment, the temperature of step (c) of the method according to the invention is above the temperature of step (d) of the method according to the invention. However, it is not excluded that the temperature of step (c) is below the temperature of step (d). In that case, the mechanism stays principally the same, but the cell attachment favoring conformation of the polymer exists at lower temperatures and changes to a non-favoring conformation at higher temperatures only.

In embodiments, in which the temperature is the medium condition to which the cells and the cell culture substrate are subjected to in step (c), the provision is that the temperature of step (c) and the temperature of step (d) are different.

In certain embodiments, it is not excluded that the temperature of step (c) and/or step (d) may be higher than 38 °C if it is allowed by the cell sample.

Temperature can be changed by placing the cell culture substrate in a temperature-controlled environment. Temperature can also be changed by adding a solution of a desired temperature into the cell or tissue culture vessel.

If the stimulus-responsive polymer layer is a pH-responsive polymer layer and/or an ionic strength-responsive polymer layer and/or a medium-responsive polymer layer, the conditions can be changed by adding a solution of a desired pH, or ionic strength, or specific medium composition into the cell or tissue culture vessel or a composition or agent that changes the pH or ionic strength of the medium to the desired value. The pH and/or ionic strength can thus also be changed by adding compositions or compounds to the medium in which the cells are grown.

Electric and magnetic fields can be changed by placing the cell culture substrate in an appropriate electric or magnetic field.

The change in adherence of the at least one cell type means that said at least one cell type loses at least part of its adherence to the polymer layer due to the change in the conditions relative to the at least one other cell type. The cells that lose adherence and the cells that maintain adherence are both part of the cell population originally immobilized on or bound to the polymer layer and differ in one or more characteristics responsible for the change in adherence upon change of medium conditions and thus of polymer properties. The loss of adherence may not be full loss in that the cells get detached, but is typically sufficient to allow their detachment in a way that allows separation from other cells less or not influenced by the change of conditions. Herein described are techniques that allow detachment and subsequently separation of those cells that are influenced by the stimulus-induced change in polymer properties in that they lose part of their adherence, such as flushing, agitation, etc. When reference is made to at least one cell type that loses adherence, this includes a fraction of the cells originally adherent to the polymer layer and may thus also comprise two or more different types of cells as long as at least one type of cells that differs from the detached cells remains adherent to the polymer layer. The same applies to the at least one cell type that remains adhered to the polymer layer and is not detached according to this method.

The term "flushing" of step (e) of the method according to the invention means rinsing the cell culture substrate, preferably with (fresh) cell culture medium or with a suitable solvent. In a preferred embodiment, cell culture medium suitable for cultivation of the cells to be separated is used, e.g. DMEM. This step may help to release at least one first type of cells with decreased adherence to the cell culture substrate. "Fresh" cell culture medium preferably means new, sterile medium that is, more preferably, free of any cells at the time point of addition.

Additionally or alternatively to step (e), agitation of the cell culture vessel after step (d) or optionally after or simultaneously with step (e) can be carried out to support the release of the at least one first type of cells with decreased adherence to the cell culture substrate. The term "agitation" may comprise shaking the vessel in any suitable way or with any suitable tool or device known to the person skilled. For example, agitation comprises tapping or knocking against the wall or slide of the cell culture vessel, for example with a finger or with a suitable object. Agitation may also comprise a suitable way of stirring within the cell culture vessel. This may support the release of at least one first type of cells, which are less adhered to the cell culture substrate after inducing the change in polymer conformation. Of course, release can further be assisted manually. Due to the risk to damage the cells or release the undesired cell type, this is however not preferred.

In various embodiments, the steps (a), and/or (b), and/or (c), and/or (d), and/or (e) of the method according to the invention are also carried out during agitation or stirring, preferably in a cell or tissue culture vessel.

Preferably, the agitation or stirring is achieved by using a method selected from a group consisting of a shaker plate, rotating orbital shaker, magnetic stir bar and stir plate, roller vessel system, and bioreactor systems with impellers and/or perfusion flow systems. In any of these embodiments, the agitation/stirring is however designed such that the adherence of the cells to the substrate before step (d), i.e. in the absence of the stimulus, is not negatively influenced, in particular such that mechanical detachment by any of the agitation devices is avoided.

In step (g) of the method according to the invention, at least one first type of cells released from the cell culture substrate is separated from at least one second type of cells that remained adhered to the cell culture substrate. Preferably, this occurs by removing the medium present in/on the cell culture substrate and discarding it or transferring it into another cell culture vessel. The remaining cell culture substrate with the cells adhered thereon is then typically contacted with fresh medium. Preferably, the cell culture substrate together with this fresh cell culture medium is subjected to conditions, which allow the separated cells to grow.

Alternatively, in step (g) of the method according to the invention, the cell culture substrate to which at least one type of cells remains adhered, can be transferred from the present cell culture vessel into a new one with fresh cell culture medium and subjected to conditions which allow the separated cells to grow.

In any case, the cells of interest may be those released and removed in step (g) or, alternatively, the ones that remain adhered to the cell culture substrate after step (g).

Preferably, the method according to the invention avoids the use of chemical or biochemical reagents, which may serve as releasing agents or digestive agents or enzymes, e.g. trypsin. Also avoided is mechanical disruption of cell adherence, such as scraping off cells. The detachment by agitation after the stimulus is however not considered "mechanical disruption".

The invention also relates to the use of the cell culture substrate comprising a temperature-responsive polymer layer as defined herein for cell separation.

All items, embodiments and examples described for the method according to the invention also apply to the use according to the invention, and vice versa.

### EXAMPLES

### Materials and methods

### Cell culture substrate

- A 5 nm thick poly(di(ethylene glycol)methyl ether methacrylate) (PDEGMA) layer was synthesized on a gold/titanium coated borosilicate solid support (20 × 20 mm², thickness 0.5 - 0.6 mm) using ω-mercaptoundecyl bromoisobutyrate as the initiator.

### Cultivation

- Cells were added to the cell culture substrate in amounts of 1.6·10⁴ cells/cm².
- Cultivation was carried out in a six-well plate or in 35 mm culture dishes in 3 mL of the respective liquid cell culture medium
- For PatuT and NIH 3T3 cells (37 °C, 24 h, 5 % CO₂)

### Media and solvents

- **PBS:** Dulbecco's phosphate saline buffer (10x, without Mg²⁺ and Ca²⁺) 2 g/L KCl, 2 g/L KH₂PO₄, 80 g/L NaCl, 21.6 g/L Na₂HPO₄*7H₂O from Lonza.
   PBS is used during cell culture substrate preparation for the fibronectin (FN) coupling step, and during analytical measurements before microscopic analysis or before RNA isolation to remove the culture media.
- **Co-culture of pancreatic cells and fibroblasts:** Dulbecco's modified Eagle medium (DMEM), 5 % fetal bovine serum, 5 % horse serum, 100 U/mL penicillin, 100 µg/mL streptomycin and 2 × 10⁻³ M L-glutamine (all from Gibco, Life technologies, Germany).
- iPS: TeSRTM medium (STEMCELL Technologies Germany GmbH, Köln, Germany).
   Media are used during cultivation, during temperature decrease, and during the releasing step.
- **Corning^{®}Matrigel^{®}Matrix:** Matrigel is a gelatinous protein mixture secreted by Engelbreth-Holm-Swarm (EHS) mouse sarcoma cells. This material, once isolated, is composed of approximately 60 % laminin, 30 % collagen IV, and 8 % entactin and polymerized at temperatures above 10 °C. The Matrigel stock solution is diluted 71 to 93-fold in 4 °C cold culture medium. Plastic tissue culture labware is covered by chilled (4 °C) liquid Matrigel solution (1 mL/9 cm²) and placed 1 h before usage at room temperature for polymerization. The remaining fluid supernatant is removed and fresh media (1 mL/9 cm²) is added on top before 1 mL/9 cm² of cells are seeded for incubation at 37 °C.
- **TCPS:** Tissue culture polystyrene: Untreated polystyrene surfaces are not suitable for cell attachment due to the surface chemistry of the polystyrene. The tissue culture treatment process involves exposing a polystyrene microplate to a plasma gas in order to modify the hydrophobic plastic surface to make it more hydrophilic. The resulting surface carries a net negative charge due to the presence of oxygen-containing functional groups such as hydroxyl and carboxyl and thus leading to increased cell attachment.

### FN coupling

- For FN coupling, the PDEGMA-coated substrates were immersed into 70 % ethanol for 1 h and rinsed twice with PBS buffer before the FN-coupling step. Then the substrates were placed in a six-well plate (standard F, Sarstedt, Kleinstadt, Germany) and incubated in a solution of FN (10 µg/mL in a solution of FN in PBS, VWR, Germany) at 37 °C for 1 h. The substrates were washed with warmed PBS and immediately used for cell seeding.

### Example 1

### Selective cell detachment of cell line co-culture (PaTu 8988t and NIH 3T3)

PaTu 8988t (pancreatic tumor) and NIH 3T3 (fibroblast) cell lines were co-cultured on a 5 nm thick PDEGMA layer. Cell attachment and proliferation of both cell lines occurred on PDEGMA at 37 °C for 24 h. Afterwards, the temperature was reduced from 37 °C to 22 °C for 30 min. Only PaTu 8988t cells showed substantial changing on cell morphology and weaker cell adherence when the environment temperature was reduced from 37 °C to 22 °C for 30 min. Then, a selective cell detachment of PaTu 8988t was obtained at 22 °C by flushing with cell culture medium (Figure 1).

### Example 2

For kinetic SPR data measurement, NIH 3T3 cell culture medium adsorption on 5 nm PDEGMA at 25 °C and 37 °C was determined (Figure 2a). Figure 2b illustrates the irreversible SPR angle shift of different cell culture medium adsorption on 5 nm PDEGMA at 25 °C and 37 °C (NIH 3T3: white, PaTu 8988t: grey).

### Example 3

Fluorescence microscopy of PaTu 8988t (Figure 3a) and NIH 3T3 (Figure 3b) cells on 5 nm PDEGMA and TCPS in different states ((i) after 24 h cultivation at standard conditions (37 °C, 5 % CO₂); (ii) after cell releasing at 22 °C, the remaining cells of PaTu 8988t and NIH 3T3 on the substrates; (iii) the released cells were reseeded on new TCPS dishes and incubated for 2 h at 37 °C). The cell nuclei were stained with Hoechst 33258 in blue. Quantitative evaluation of PaTu 8988t (Figure 3c) and NIH 3T3 (Figure 3d) cell numbers at 37 °C after 24 h cultivation and after 30 min incubation at 22 °C and cell medium washing step on the substrates as well as detached cells reseeded on new TCPS dishes (grey: TCPS, white: 5 nm PDEGMA), where error bars are standard error (n = 3), ns means not significant, indicating p > 0.05, * p < 0.05, and ** p < 0.0001.

### Example 4

Statistical analysis of NIH 3T3 (grey) and PaTu 8988t (white) co-cultures on (a) 5 nm PDEGMA layer and (b) TCPS in the attached and the released state. PaTu 8988t cells were stained with Vybrant^{®} DiD (red), and NIH 3T3 cells were labelled with Vybrant^{®} DiO (green). The samples were quantitatively analyzed by fluorescence microscopy. The error bars are the standard deviation from 3 different images per condition. According to Figure 4, where the fraction of two cell lines in the different states on PDEGMA and TCPS are compared, there was a significant difference in the composition of the co-culture. There were 95 ± 6 % of PaTu 8988t cells detached from PDEGMA layer (Figure 4a), whereas only few cells were released from TCPS (9 ± 2 %, Figure 4b). By contrast, a small amount of NIH 3T3 fibroblasts detached from PDEGMA and TCPS surfaces, 13 ± 1 % and 8 ± 4 %. The observations confirm that 5 nm thin PDEGMA layers enable to release PaTu 8988t cells, preferably from a co-culture with NIH 3T3 cells, and thus afford a strategy for selective cell detachment and thus purification.

### Example 5

### Propagation and purification of human induced pluripotent stem (iPS) cells

Human induced pluripotent stem (iPS) cells were seeded on 5 nm PDEGMA brushes. Cells grew into cell colony in 5 days incubation at 37 °C. The undifferentiated iPS cell colony detached from the heterogeneous culture, while the differentiated cells remained on the PDEGMA brushes at 22 °C (Figure 5).

### Example 6

iPS cells were cultured in colonies on 5 nm PDEGMA brushes for 5 days at 37 °C (Figure 6a). Subsequently, the temperature was reduced to 22 °C and iPS cell colonies detached from the 5 nm PDEGMA brushes after 135 min (Figure 6b), and after 345 min (Figure 6c). Figures 6a-c are optical microscopy images. Figure 6d illustrates the time needed for reducing the surface area of different iPS colonies on 5 nm PDEGMA brushes at 22 °C.

### Example 7

iPS colony was cultured on matrigel at 37 °C (Figure 7a). Morphology changing of the iPS colony on matrigel at 22 °C after 360 min are illustrated in Figure 7b. Figures 7a-b are optical microscopy images. Figure 7c shows the time needed for reducing the surface area of different iPS colonies on matrigel at 22 °C.

### Example 8

iPS cells were cultured in colonies with surrounding differentiated cells on 5 nm PDEGMA layer for 5 days at 37 °C (Figure 8a). Selective detachment of undifferentiated iPS cell colonies from 5 nm PDEGMA layer at 22 °C were illustrated in Figure 8b after 120 min and in Figure 8c after 210 min. Figure 8d illustrates the released iPS cell colonies floating in the cell culture medium after knocking the culture plate. Remaining differentiated cells on the substrate after replacing the culture plate at 37 °C for 60 min was demonstrated in Figure 8e. Figures 8a-e are optical microscopy images. Figure 8f illustrates the results of semi-quantitative reverse transcriptase PCR of iPS cells cultured on (A) matrigel and (B) 5 nm PDEGMA for 5 days, (C) released iPS cells from PDEGMA layer by the thermal stimulus. The gene expression level of pluripotency markers Sox 2, Oct 3/4, c-Myc confirmed the pluripotency maintenance of iPS cells grown on 5 nm PDEGMA as well as released iPS cells compared to the standard growth conditions on matrigel. GAPDH as a housekeeping gene serves as an internal reference gene for normalization.

## Claims

1. . A method for separating cells, comprising or consisting of the following steps:
(a) providing a sample containing at least two different types of cells;
(b) contacting the sample with a cell culture substrate comprising a temperature-responsive polymer layer, wherein the temperature-responsive polymer layer exhibits a lower critical solution temperature (LCST) of 10 to 50 °C;
(c) subjecting the cell culture substrate and the cells to medium conditions comprising temperature conditions which allow the two different types of cells to adhere to the cell culture substrate;
(d) modifying the temperature conditions of step (c) to decrease adherence of at least one first type of cells to the cell culture substrate, wherein the adherence of at least one second type of cells remains essentially unaffected;
(e) optionally flushing the cell culture substrate of step (d) with a suitable cell culture medium or solvent to support the release of the at least one first type of cells with decreased adherence to the cell culture substrate;
(f) optionally agitating the cell culture vessel after step (d) or optionally after step (e) to support the release of the at least one first type of cells with decreased adherence to the cell culture substrate;
(g) separating the at least one first type of cells released from the cell culture substrate in step (d) and/or (e) and/or (f) from the at least one second type of cells that remain adhered to the cell culture substrate.

2. . The method according to claim 1 , wherein the medium conditions that are modified in step (d) further comprise pH, ionic strength, solvent, cell culture medium, salt, surfactant, and/or electric or magnetic field conditions.

3. . The method according to claim 1 or 2, wherein the cell culture substrate and the cells of step (c) are subjected to medium conditions comprising a temperature of 2 °C to 38 °C, preferably of 10 °C to 38 °C, more preferably of 20 °C to 38 °C, more preferably of 28 °C to 38 °C, more preferably of 35 °C to 37.5 °C, most preferably about 37 °C.

4. . The method according to any of claims 1 to 3, wherein the cell culture substrate and the cells of step (c) are subjected to said medium conditions for 1 minute up to 10 days, preferably for 1 hour up to 6 days, more preferably for about 24 hours, or about 48 hours, or about 5 days.

5. . The method according to any of claims 1 to 4, wherein modifying the medium conditions of step (d) comprises a temperature reduction by at least 2 °C, preferably by at least 5 °C, more preferably by at least 8 °C, more preferably by at least 12 °C, and most preferably by at least 15 °C.

6. . The method according to any of claims 1 to 5, wherein the cell culture substrate and the cells of step (d) are subjected to the modified medium conditions for 1 minute up to 10 hours, preferably for 15 minutes up to 6 hours.

7. . The method according to any of claims 1 to 6, wherein the cell culture substrate comprises a solid support, preferably coated with a metal, wherein the metal is preferably selected from gold, titanium, or an alloy comprising one or both thereof.

8. . The method according to any of claims 1 to 7, wherein the solid support comprises or consists of a material selected from the group consisting of glass, silicone, polymers, e.g. polystyrene, polymethylmethacrylate, or polycarbonate; ceramic, metals, metal or semiconductor oxides, inorganic solids, or a mixture thereof.

9. . The method according to any of claims 1 to 8, wherein the binding of the temperature-responsive polymer layer to the solid support is facilitated by an initiator compound.

10. . The method according to any of claims 1 to 9, wherein the temperature-responsive polymer layer is generated in situ by surface-initiating atom transfer radical polymerization starting from an initiator compound coupled to the solid support.

11. . The method according to any of claims 1 to 10, wherein the temperature-responsive polymer layer
(1) comprises homopolymers, preferably selected from the group consisting of methacrylate homopolymers, more preferably poly(di(ethylene glycol)methyl ether methacrylate) homopolymers; and/or
(2) has a dry ellipsometric thickness of 1 to 1000 nm, preferably of 1 .5 to 100 nm, more preferably of 2 to 25 nm, more preferably of 3 to 10 nm, most preferably of about 5 nm, preferably at 25 °C measured by an ellipsometer in air, atomic force microscopy (AFM), interferometry, surface plasmon resonance (SPR), X-Ray Photoelectron Spectroscopy (XPS), and/or by Fourier-Transform Infrared Spectroscopy (FTIR); and/or
(3) exhibits a lower critical solution temperature (LCST) of 12 to 45 °C, preferably of 15 to 40 °C, more preferably of 20 to 37 °C, more preferably of 25 to 36 °C, most preferably of 32 to 35 °C, preferably in a suitable cell culture medium.

12. . The method according to any of claims 1 to 11, wherein the cells to be separated are mixtures of
(i) different cell lines, preferably tumor cells and fibroblast cells; or
(ii) pathologically altered cells and normal cells; or
(iii) differentiated stem cells and undifferentiated stem cells, preferably mixtures of undifferentiated human induced pluripotent stem cells and differentiated cells; or
(iv) different cell types derived from a certain tissue (e.g. from biopsies).

13. . The method according to any of claims 1 to 12, wherein the cells to be separated are added to the cell culture substrate in amounts of 1 ,000 to 100,000 cells/cm² , preferably of 10,000 to 40,000 cells/cm² , more preferably of 15,000 to 20,000 cells/cm².

14. . Use of a cell culture substrate comprising a temperature-responsive polymer layer as defined in any of claims 1 to 13 for cell separation.

## Patentansprüche

1. Verfahren zum Trennen von Zellen, umfassend oder bestehend aus den folgenden Schritten:
(a) Bereitstellen einer Probe, die mindestens zwei verschiedene Typen von Zellen enthält;
(b) Inkontaktbringen der Probe mit einem Zellkultursubstrat umfassend eine Temperatur-responsive Polymerschicht, wobei die Temperatur-responsive Polymerschicht eine untere kritische Lösungstemperatur (LCST) von 10 bis 50 °C aufweist;
(c) Aussetzen des Zellkultursubstrats und der Zellen gegenüber Mediumbedingungen umfassend Temperaturbedingungen, welche es den zwei verschiedenen Typen von Zellen ermöglichen, an dem Zellkultursubstrat zu haften;
(d) Modifizieren der Temperaturbedingungen von Schritt (c), um die Adhäsion mindestens eines ersten Zelltyps an das Zellkultursubstrat zu verringern, wobei die Adhäsion mindestens eines zweiten Zelltyps im Wesentlichen unbeeinflusst bleibt;
(e) gegebenenfalls Spülen des Zellkultursubstrats von Schritt (d) mit einem geeigneten Zellkulturmedium oder Lösungsmittel, um die Freisetzung des mindestens einen ersten Zelltyps mit verringerter Adhäsion an das Zellkultursubstrat zu unterstützen;
(f) gegebenenfalls Schütteln des Zellkulturgefäßes nach Schritt (d) oder gegebenenfalls nach Schritt (e), um die Freisetzung des mindestens einen ersten Zelltyps mit verringerter Adhäsion an das Zellkultursubstrat zu unterstützen;
(g) Trennen des mindestens einen ersten Zelltyps, welcher in Schritt (d) und/oder (e) und/oder (f) von dem Zellkultursubstrat freigesetzt wurde, von dem mindestens einen zweiten Zelltyp, der an dem Zellkultursubstrat haften bleibt.

2. Das Verfahren gemäß Anspruch 1, wobei die in Schritt (d) modifizierten Mediumbedingungen des Weiteren pH-Wert, Ionenstärke, Lösungsmittel, Zellkulturmedium, Salz, Tensid und/oder elektrische oder magnetische Feldbedingungen umfassen.

3. Das Verfahren gemäß Anspruch 1 oder 2, wobei das Zellkultursubstrat und die Zellen von Schritt (c) Mediumbedingungen ausgesetzt werden, die eine Temperatur von 2 °C bis 38 °C, vorzugsweise von 10 °C bis 38 °C, bevorzugter von 20 °C bis 38 °C, noch bevorzugter von 28 °C bis 38 °C, noch bevorzugter von 35 °C bis 37, 5 °C, am meisten bevorzugt ungefähr 37 ° umfassen.

4. Das Verfahren gemäß einem der Ansprüche 1 bis 3, wobei das Zellkultursubstrat und die Zellen von Schritt (c) den Mediumbedingungen für 1 Minute bis zu 10 Tagen, vorzugsweise für 1 Stunde bis zu 6 Tagen, bevorzugter für ungefähr 24 Stunden oder ungefähr 48 Stunden oder ungefähr 5 Tage ausgesetzt werden.

5. Das Verfahren gemäß einem der Ansprüche 1 bis 4, wobei das Modifizieren der Mediumbedingungen von Schritt (d) eine Temperaturreduktion um mindestens 2 °C, vorzugsweise um mindestens 5 °C, bevorzugter um mindestens 8 °C, noch bevorzugter um mindestens 12 °C und am meisten bevorzugt um mindestens 15 °C umfasst.

6. Das Verfahren gemäß einem der Ansprüche 1 bis 5, wobei das Zellkultursubstrat und die Zellen von Schritt (d) den modifizierten Mediumbedingungen für 1 Minute bis zu 10 Stunden, vorzugsweise für 15 Minuten bis zu 6 Stunden ausgesetzt werden.

7. Das Verfahren gemäß einem der Ansprüche 1 bis 6, wobei das Zellkultursubstrat einen festen Träger umfasst, vorzugsweise beschichtet mit einem Metall beschichtet, wobei das Metall vorzugsweise ausgewählt ist aus Gold, Titan oder einer Legierung umfassend eines oder beide davon.

8. Das Verfahren gemäß einem der Ansprüche 1 bis 7, wobei der feste Träger ein Material umfasst oder daraus besteht, welches ausgewählt ist aus der Gruppe bestehend Glas, Silikon, Polymeren, beispielsweise Polystyrol, Polymethylmethacrylat oder Polycarbonat; Keramik, Metallen, Metall- oder Halbleiteroxiden, anorganischen Feststoffen oder einer Mischung davon.

9. Das Verfahren gemäß einem der Ansprüche 1 bis 8, wobei die Bindung der Temperatur-responsiven Polymerschicht an den festen Träger durch eine Initiatorverbindung erleichtert wird.

10. Das Verfahren gemäß einem der Ansprüche 1 bis 9, wobei die Temperatur-responsive Polymerschicht in situ durch eine oberflächeninitiierende radikalische Atomtransferpolymerisation erzeugt wird, die von einer an den festen Träger gekoppelten Initiatorverbindung ausgeht.

11. Das Verfahren gemäß einem der Ansprüche 1 bis 10, wobei die Temperatur-responsive Polymerschicht
(1) Homopolymere umfasst, vorzugsweise ausgewählt aus der Gruppe bestehend aus Methacrylat-Homopolymeren, bevorzugter Poly(di(ethylenglycol)methylethermethacrylat)-Homopolymeren; und/oder
(2) eine trockene ellipsometrische Dicke von 1 bis 1000 nm, vorzugsweise von 1,5 bis 100 nm, bevorzugter von 2 bis 25 nm, noch bevorzugter von 3 bis 10 nm, am meisten bevorzugt von ungefähr 5 nm, vorzugsweise bei 25 °C, gemessen durch ein Ellipsometer in Luft, Rasterkraftmikroskopie (AFM), Interferometrie, Oberflächenplasmonenresonanz (SPR), Röntgen-Photoelektronenspektroskopie (XPS) und/oder durch Fourier-Transform-Infrarotspektroskopie (FTIR); und/oder
(3) eine niedrigere kritische Lösungstemperatur (LCST) von 12 bis 45 °C, vorzugsweise von 15 bis 40 °C, bevorzugter von 20 bis 37 °C, noch bevorzugter von 25 bis 36 °C, am meisten bevorzugt von 32 bis 35 °C, vorzugsweise in einem geeigneten Zellkulturmedium, aufweist.

12. Das Verfahren gemäß einem der Ansprüche 1 bis 11, wobei die zu trennenden Zellen Mischungen sind aus
(i) unterschiedlichen Zelllinien, vorzugsweise Tumorzellen und Fibroblastenzellen; oder
(ii) pathologisch veränderten Zellen und normalen Zellen; oder
(iii) differenzierten Stammzellen und undifferenzierten Stammzellen, vorzugsweise Mischungen aus undifferenzierten humanen induzierten pluripotenten Stammzellen und differenzierten Zellen; oder
(iv) unterschiedlichen Zelltypen, die von einem bestimmten Gewebe (beispielsweise von Biopsien) stammen.

13. Das Verfahren gemäß einem der Ansprüche 1 bis 12, wobei die zu trennenden Zellen dem Zellkultursubstrat in Mengen von 1000 bis 100000 Zellen/cm², vorzugsweise von 10000 bis 40000 Zellen/cm², bevorzugter von 15000 bis 20000 Zellen/cm² zugesetzt werden.

14. Verwendung eines Zellkultursubstrats umfassend eine Temperatur-responsive Polymerschicht, wie in einem der Ansprüche 1 bis 13 definiert, zur Zelltrennung.

## Revendications

1. Procédé de séparation de cellules, comprenant ou consistant en les étapes suivantes :
(a) mise à disposition d'un échantillon contenant au moins deux types de cellules différents ;
(b) mise en contact de l'échantillon avec un substrat de culture cellulaire comprenant une couche de polymère sensible à la température, sachant que la couche de polymère sensible à la température présente une température critique inférieure de solution (LCST) de 10 à 50 °C ;
(c) soumission du substrat de culture cellulaire et des cellules à des conditions de milieu comprenant des conditions de température qui permettent aux deux types de cellules différents d'adhérer au substrat de culture cellulaire ;
(d) modification des conditions de température de l'étape (c) pour réduire l'adhérence d'au moins un premier type de cellules au substrat de culture cellulaire, sachant que l'adhérence d'au moins un deuxième type de cellules reste sensiblement inchangée ;
(e) facultativement, rinçage du substrat de culture cellulaire de l'étape (d) avec un milieu de culture cellulaire ou un solvant adéquat pour favoriser le détachement de l'au moins un premier type de cellules à adhérence réduite au substrat de culture cellulaire ;
(f) facultativement, agitation du récipient de culture cellulaire après l'étape (d) ou facultativement après l'étape (e) pour favoriser le détachement de l'au moins un premier type de cellules à adhérence réduite au substrat de culture cellulaire ;
(g) séparation de l'au moins un premier type de cellules détachées du substrat de culture cellulaire à l'étape (d) et/ou (e) et/ou (f) de l'au moins un deuxième type de cellules qui continuent d'adhérer au substrat de culture cellulaire.

2. Le procédé selon la revendication 1, sachant que les conditions de milieu qui sont modifiées à l'étape (d) comprennent en outre le pH, la force ionique, le solvant, le milieu de culture cellulaire, le sel, le tensioactif, et/ou les conditions électriques et/ou de champ magnétique.

3. Le procédé selon la revendication 1 ou 2, sachant que le substrat de culture cellulaire et les cellules de l'étape (c) sont soumis à des conditions de milieu comprenant une température de 2 °C à 38 °C, de préférence de 10 °C à 38 °C, de manière plus préférentielle de 20 °C à 38 °C, de manière plus préférentielle de 28 °C à 38 °C, de manière plus préférentielle de 35 °C à 37,5 °C, de manière la plus préférentielle d'environ 37 °C.

4. Le procédé selon l'une quelconque des revendications 1 à 3, sachant que le substrat de culture cellulaire et les cellules de l'étape (c) sont soumis auxdites conditions de milieu pendant 1 minute à 10 jours, de préférence pendant 1 heure à 6 jours, de manière plus préférentielle pendant environ 24 heures, ou environ 48 heures, ou environ 5 jours.

5. Le procédé selon l'une quelconque des revendications 1 à 4, sachant que la modification des conditions de milieu de l'étape (d) comprend une réduction de température d'au moins 2 °C, de préférence d'au moins 5 °C, de manière plus préférentielle d'au moins 8 °C, de manière plus préférentielle d'au moins 12 °C, et de manière la plus préférentielle d'au moins 15 °C.

6. Le procédé selon l'une quelconque des revendications 1 à 5, sachant que le substrat de culture cellulaire et les cellules de l'étape (d) sont soumis aux conditions de milieu modifiées pendant 1 minute à 10 heures, de préférence pendant 15 minutes à 6 heures.

7. Le procédé selon l'une quelconque des revendications 1 à 6, sachant que le substrat de culture cellulaire comprend un support solide, de préférence revêtu d'un métal, sachant que le métal est de préférence sélectionné parmi l'or, le titane, ou un alliage comprenant l'un ou les deux de ceux-ci.

8. Le procédé selon l'une quelconque des revendications 1 à 7, sachant que le support solide comprend ou est composé d'un matériau sélectionné dans le groupe constitué par le verre, le silicone, les polymères, par exemple le polystyrène, le polyméthylméthacrylate, ou le polycarbonate ; la céramique, les métaux, les oxydes métalliques ou semiconducteurs, les solides inorganiques, ou un mélange de ceux-ci.

9. Le procédé selon l'une quelconque des revendications 1 à 8, sachant que la liaison de la couche de polymère sensible à la température au support solide est facilitée par un composé initiateur.

10. Le procédé selon l'une quelconque des revendications 1 à 9, sachant que la couche de polymère sensible à la température est générée in situ par polymérisation radicalaire par transfert d'atome d'initiation à la surface partant d'un composé initiateur couplé au support solide.

11. Le procédé selon l'une quelconque des revendications 1 à 10, sachant que la couche polymère sensible à la température
(1) comprend des homopolymères, de préférence sélectionnés dans le groupe constitué par des homopolymères de méthacrylate, de manière plus préférentielle des poly(di(éthylène glycol)méthyl éther méthacrylate) homopolymères ; et/ou
(2) a une épaisseur ellipsométrique à sec de 1 à 1 000 nm, de préférence de 1,5 à 100 nm, de manière plus préférentielle de 2 à 25 nm, de manière plus préférentielle de 3 à 10 nm, de manière la plus préférentielle d'environ 5 nm, de préférence à 25 °C, mesurée par un ellipsomètre dans l'air, microscopie à force atomique (AFM), interférométrie, résonance plasmonique de surface (SPR), spectroscopie photoélectronique X (XPS), et/ou spectroscopie infrarouge à transformée de Fourier (FTIR) ; et/ou
(3) présente une température critique inférieure de solution (LCST) de 12 à 45 °C, de préférence de 15 à 40 °C, de manière plus préférentielle de 20 à 37 °C, de manière plus préférentielle de 25 à 36 °C, de manière la plus préférentielle de 32 à 35 °C, de préférence dans un milieu de culture cellulaire adéquat.

12. Le procédé selon l'une quelconque des revendications 1 à 11, sachant que les cellules à séparer sont des mélanges de
(i) différentes lignes cellulaires, de préférence des cellules tumorales ou des cellules de fibroblastes ; ou
(ii) des cellules altérées pathologiquement et des cellules normales ; ou
(iii) des cellules souches différenciées et des cellules souches indifférenciées, de préférence des mélanges de cellules souches pluripotentes induites humaines indifférenciées et des cellules différenciées ; ou
(iv) différents types de cellules dérivées d'un certain tissu (par exemple de biopsies).

13. Le procédé selon l'une quelconque des revendications 1 à 12, sachant que les cellules à séparer sont ajoutées au substrat de culture cellulaire dans des quantités de 1 000 à 100 000 cellules/cm², de préférence de 10 000 à 40 000 cellules/cm², de manière plus préférentielle de 15 000 à 20 000 cellules/cm².

14. Utilisation d'un substrat de culture cellulaire comprenant une couche de polymère sensible à la température telle que définie dans l'une quelconque des revendications 1 à 13 pour la séparation de cellules.
